# EUROPEAN PATENT APPLICATION

(11) **EP 1 447 662 A1**
(43) Date of publication of application: **18.08.2004**
(21) Application number: 02781789.9
(22) Date of filing: 15.11.2002
(51) Int. Cl.: G01N 30/14, G01N 30/72, G01N 30/64, G01N 30/88, G01N 27/06, G01N 27/62

(54) **METHOD OF DETERMINING CATIONIC SURFACTANT**

(30) Priority: 22.11.2001 JP 2001356859
(71) Applicant: Nihon University, Tokyo 102-0074 (JP)
(72) Inventor: SHIBUKAWA, Masami, C/O Nihon University, Tokyo 102-0074 (JP)
(74) Representative: Hartz, Nikolai F., Dr.
(86) International application number: PCT/JP2002/011937
(87) International publication number: WO 2003/044518

(57) **Abstract**

A method of determining a cationic surfactant in a sample solution, characterized by passing the sample solution through a disk containing styrene/divinylbenzene copolymer particles and a disk containing an anion-exchange resin; and then subjecting the resultant solution to high-performance liquid chromatography.

The method of the present invention enables separation/quantification of CS in river water or lake water which also contains inorganic ions and AS of high concentration, even when the CS content is below the order of ppb.

## Description

### Technical Field

The present invention relates to a method for correctly and rapidly determining cationic surfactants in an aqueous solution such as environmental water, even when the cationic surfactant coexists with an anionic surfactant in the solution; and to an apparatus employed in the method.

### Background Art

Cationic surfactants (CS) have widely been employed as primary components of, for example, a hair rinse, a hair treatment agent, or a softening agent for clothing. However, since CS is not rapidly decomposed by microorganisms because of its sterilizability, concern has arisen about the effect of CS on aquatic living organisms. Accordingly, determination of CS in environmental water is very important, and development of a method for rapid analysis of low-concentration CS is demanded.

In order to establish a highly sensitive separation/quantification method for CS in environmental water by means of high-performance liquid chromatography (HPLC) employing an electrical conductivity detector, the present inventors have conducted studies on HPLC systems employing a hydrophilic polymer gel column serving as a separation column. As a result, the present inventors have developed a highly sensitive HPLC system which provides a detection limit of 0.03 to 0.04 µM, which is on the order of 1/10 or less compared with that of a conventional HPLC system employing an electrical conductivity detector or an indirect UV absorption detector.

However, the concentration of CS present in river water is considered to be on the order of ppb or less, and therefore, determining the CS content requires concentration of CS in a sample and removal of inorganic ions and anionic surfactants (AS).

In view of the foregoing, an object of the present invention is to provide a method for accurately determining the CS concentration in an aqueous solution containing both AS and CS such as environmental water.

### Disclosure of the Invention

In order to attain the above object, the present inventors have performed extensive studies on disks containing a variety of resins that can separate AS from CS. As a result, they have quite unexpectedly found that, when a disk containing styrene-divinylbenzene copolymer particles is employed in combination with a disk containing an anion-exchange resin, AS is selectively removed from the solution and CS is concentrated, and that when the resultant solution is subjected to solid-phase extraction, if necessary, and then subjected to HPLC, the amount of CS can be accurately determined. The present invention has been accomplished on the basis of these findings.

Accordingly, the present invention provides a method for quantifying cationic surfactants in a sample solution, which comprises causing the sample solution to pass through a disk containing styrene-divinylbenzene copolymer particles and a disk containing an anion-exchange resin; and subsequently subjecting the resultant solution to high-performance liquid chromatography.

The present invention also provides a method for quantifying a cationic surfactant in a sample solution, which method comprises causing the sample solution to pass through a disk containing styrene-divinylbenzene copolymer particles and a disk containing an anion-exchange resin, and through a solid-phase extraction column; and subsequently subjecting the resultant solution to high-performance liquid chromatography.

The present invention also provides an apparatus for measuring a cationic surfactant in a sample solution, the apparatus comprising a disk containing styrene-divinylbenzene copolymer particles, a disk containing an anion-exchange resin, and a high-performance liquid chromatograph.

The present invention also provides an apparatus for measuring a cationic surfactant in a sample solution, the apparatus comprising a disk containing styrene-divinylbenzene copolymer particles, a disk containing an anion-exchange resin, a solid-phase extraction column, and a high-performance liquid chromatograph.

### Brief Description of the Drawings

Fig. 1 is a schematic representation showing an on-line solid-phase extraction/HPLC system employed in the present invention.
Fig. 2 shows the results of separation of CS in a river water sample, the CS separation having been performed through preliminary treatment by use of SDB-XD (disk containing SDB particles) and ANION-SR (disk containing anion-exchange resin), on-line solid-phase extraction, and HPLC.
Fig. 3 shows the results of standard addition test employing CTMA (cetyltrimethylammonium ion) and TMSA (trimethylstearylammonium ion) in separation/quantification of CS in a river water sample, the CS separation/quantification having been performed through preliminary treatment by use of SDB-XD and ANION-SR, on-line solid-phase extraction, and HPLC.

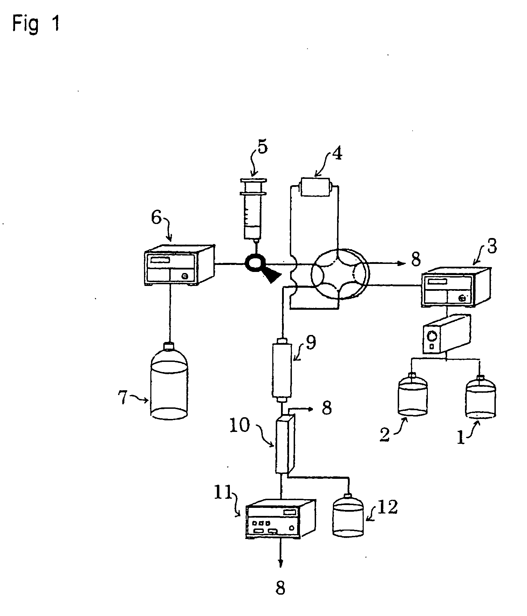

Reference numerals shown in Fig. 1 denote the following items.
- 1:: Sample solution
- 2:: Pure water
- 3:: Pump
- 4:: Solid-phase extraction column
- 5:: Syringe
- 6:: Pump
- 7:: Eluent
- 8:: Waste
- 9:: Separation column
- 10:: Suppressor
- 11:: Detector
- 12:: Regeneration solution

### Best Mode for Carrying Out the Invention

No particular limitations are imposed on the sample solution employed in the quantifying method of the present invention, so long as the sample solution is an aqueous solution containing CS. Specific examples of the sample solution include environmental water such as river water or lake water. There has not yet been developed a suitable method for determining CS in river water or lake water, which contains, in addition to CS, large amounts of AS and inorganic ions.

In the method of the present invention, firstly, a sample solution is caused to pass through a disk containing styrene-divinylbenzene copolymer (SDB) particles and a disk containing an anion-exchange resin. When the sample solution is caused to pass through these disks, AS contained in the solution is removed, and CS is selectively collected. The finding that merely a combination of the above two disks, among numerous disks, enables removal of AS and high-efficiency collection of CS is a quite unexpected one. Each of these disks is prepared by fixing filler particles onto fibrous matter, which preferably comprises a fluorine-containing resin, such as Teflon (registered trademark)'. The anion-exchange resin is preferably a quaternary-ammonium-salt-containing anion-exchange resin. These disks may be Empore Disk SDB-XD and Empore Disk ANION-SR (trade names), which are available from 3M. No particular limitations are imposed on the sequence in which these disks are used.

Subsequently, the sample which has passed through these disks is, if necessary, caused to pass through a solid-phase extraction column, and then subjected to high-performance liquid chromatography (HPLC). When the sample is subjected to solid-phase extraction, CS in the sample can be highly concentrated, and sensitivity for determining the CS content by means of HPLC is drastically enhanced. The CS concentration is preferably carried out by means of on-line solid-phase extraction.

As shown in Fig. 1, on-line solid-phase extraction is carried out by supplying pure water 2 and a sample solution 1 to a solid-phase extraction column 4 by use of a pump 3, and by subsequently circulating the sample solution through the column a predetermined number of times. The solid-phase extraction employs a solid phase filled with a hydrophilic polymer; for example, a solid phase filled with a highly cross-linked polyvinyl alcohol gel. Examples of the polyvinyl-alcohol-gel-filled solid phase include Shodex Asahipak GF-310HQ (Showa Denko K.K.).

The sample which has undergone the CS concentration or the sample which has passed through the aforementioned disks is subjected to HPLC. As shown in Fig. 1, the system for HPLC includes a separation column 9, a suppressor 10, and a detector 11. The separation column is preferably a column filled with a hydrophilic polymer; for example, a column filled with a highly cross-linked polyvinyl alcohol gel. Specific examples of the column include Shodex Asahipak GF-310HQ (Showa Denko K.K.). Examples of the suppressor to be employed include a suppressor employing an anion-exchange membrane, such as DIONEX CMMS-II (Nippon Dionex K.K.).

Examples of the detector 11 include an electrical conductivity detector, an indirect UV absorption detector, and a mass spectrometric detector. The detector 11 is particularly preferably an electrical conductivity detector or a mass spectrometric detector. In the case where a mass spectrometric detector is employed, even when solid-phase extraction is not carried out before subjecting HPLC, CS of very low concentration can be quantified. Meanwhile, in the case where an electrical conductivity detector is employed, preferably, solid-phase extraction is carried out before subjecting HPLC.

The method of the present invention can determine the CS level of river water or lake water which also contains AS in an amount more than 100 times that of the CS, even when the CS content is below the order of ppb.

### Example

The present invention will next be described in more detail by way of Example, which should not be construed as limiting the invention thereto.

### Example 1

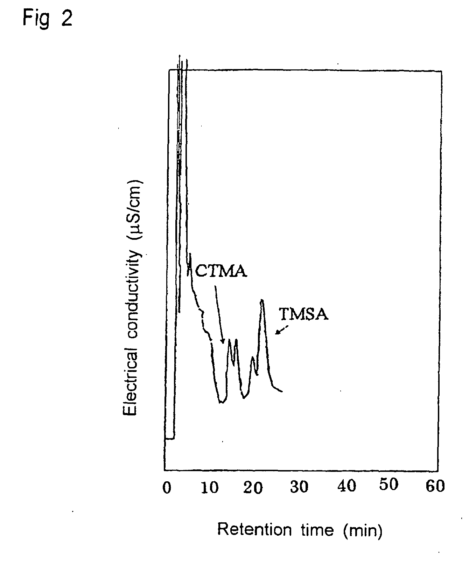

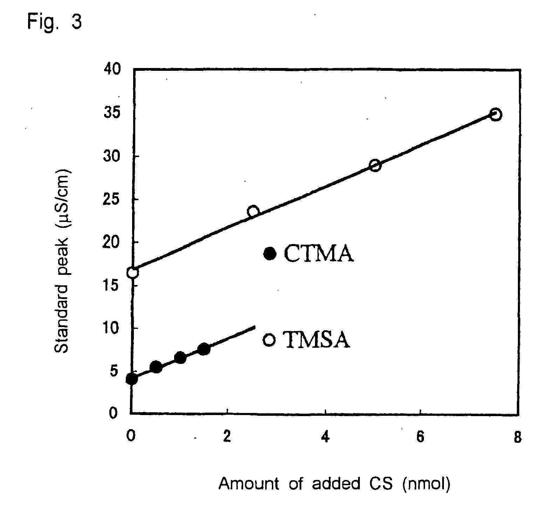

(1) An on-line solid-phase extraction/HPLC system as shown in Fig. 1 was employed. A solution mixture of 4,4'-dipyridyl, hydrochloric acid, and acetonitrile was employed as an eluent 7, and Shodex Asahipak GF-310HQ (4.6 mm I.D. × 150 mm, 6 µm) was employed as a separation column 9. As a solid-phase extraction column 4, there was employed a column (4.6 mm I.D. × 10 mm, 9 µm) filled with a filler which is formed of the same base material as that of the filler of the separation column, but has a particle size different from that of the separation column filler. DIONEX CMMS-II (Nippon Dionex K.K.) was employed as a suppressor 10, and a tetrabutylammonium hydroxide solution was employed as a regeneration solution 12. As a sample 1, the following three cation species were employed: cetyltrimethylammonium (CTMA) ion, tetradecyldimethylbenzylammonium (TDDBA) ion, and trimethylstearylammonium (TMSA) ion. The following types of Empore Disk were employed: SDB-XD (disk containing SDB particles), CATION-SR (disk containing cation-exchange resin), ANION-SR (disk containing anion-exchange resin), and SDB-RPS (disk containing sulfonated SDB particles).
(2) Firstly, the CS concentration was regulated to 50 nM, and collection/concentration of CS by Empore Disk was evaluated. Specifically, the sample solution was caused to pass through each of the above disks, and then the CS content of the resultant solution was determined by use of the on-line solid-phase extraction/HPLC system shown in Fig. 1. In order to prevent adsorption of the CS onto the wall of a glass container, hydrochloric acid was added to the sample solution such that the hydrochloric acid concentration became 0.1 M. The results of the CS content determination revealed that, in terms of CS collection efficiency, SDB-RPS is superior to SDB-XD, and SDB-XD is superior to ANION-SR. However, when AS (5 µM) was added to the sample solution, each of the disks exhibited considerably lowered percent collection of the CS. In an effort to attain high percent collection of the CS, extensive studies were performed. The results of the studies revealed that when SDB-XD (disk containing SDB particles) is stacked on ANION-SR (disk containing anion-exchange resin), and the sample solution is caused to pass through the thus-stacked disks, AS is removed, and the percent collection of the CS becomes high (95% or more). Such high percent collection was not attained by means of other combinations of the disks. Even when SDB-XD and ANION-SR were stacked in an inverse manner, similar effects were obtained.
(3) On the basis of the results of (2) above, the method of the present invention was applied in practice to river water. Fig. 2 shows a chromatogram of a river water sample collected from the Ebi River flowing through Funabashi City. CS in the sample was identified through overlap injection, since the retention time of the CS is affected by substances coexisting with the CS. As s result, CTMA and TMSA were found to be present in the sample. Mass spectrometry of the sample also confirmed the presence of CTMA and TMSA. When the sample was subjected to standard addition test for determining the amounts of these CSs, good linear relations were obtained as shown in Fig. 3. On the basis of the results, the amounts of CTMA and TMSA contained in the river water sample were calculated as 1.7 nM (0.54 ppb) and 7.0 nM (2.5 ppb), respectively.

### Industrial Applicability

The method of the present invention enables separation/quantification of CS in river water or lake water which also contains inorganic ions and AS of high concentration, even when the CS content is below the order of ppb.

## Claims

1. A method for quantifying a cationic surfactant in a sample solution, which method comprises causing the sample solution to pass through a disk containing styrene-divinylbenzene copolymer particles and a disk containing an anion-exchange resin; and subsequently subjecting the resultant solution to high-performance liquid chromatography.

2. A quantifying method according to claim 1, wherein the sample solution is an aqueous solution containing a cationic surfactant and an anionic surfactant.

3. A quantifying method according to claim 1 or 2, wherein detection means employed in the high-performance liquid chromatography is mass spectrometry.

4. A method for quantifying a cationic surfactant in a sample solution, which method comprises causing the sample solution to pass through a disk containing styrene-divinylbenzene copolymer particles and a disk containing an anion-exchange resin, and through a solid-phase extraction column; and subsequently subjecting the resultant solution to high-performance liquid chromatography.

5. A quantifying method according to claim 4, wherein the sample solution is an aqueous solution containing a cationic surfactant and an anionic surfactant.

6. A quantifying method according to claim 4 or 5, wherein detection means employed in the high-performance liquid chromatography is electrical conductivity analysis or mass spectrometry.

7. An apparatus for measuring a cationic surfactant in a sample solution, the apparatus comprising a disk containing styrene-divinylbenzene copolymer particles, a disk containing an anion-exchange resin, and a high-performance liquid chromatograph.

8. An apparatus for measuring a cationic surfactant in a sample solution, the apparatus comprising a disk containing styrene-divinylbenzene copolymer particles, a disk containing an anion-exchange resin, a solid-phase extraction column, and a high-performance liquid chromatograph.
